(19) **Europäisches Patentamt / European Patent Office / Office européen des brevets**

(11) **EP 3 896 098 A1**

(12) # EUROPEAN PATENT APPLICATION
published in accordance with Art. 153(4) EPC

(43) Date of publication:
**20.10.2021 Bulletin 2021/42**

(21) Application number: **19897219.2**

(22) Date of filing: **12.12.2019**

(51) Int Cl.:
*C08F 20/06* (2006.01)      *A61F 13/53* (2006.01)
*B01J 20/26* (2006.01)      *B01J 20/28* (2006.01)

(86) International application number:
**PCT/JP2019/048794**

(87) International publication number:
**WO 2020/122201 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2018   JP 2018232847**
         **22.03.2019   JP 2019054977**

(71) Applicant: **SUMITOMO Seika Chemicals Co., Ltd.**
**Kako-gun, Hyogo 675-0145 (JP)**

(72) Inventor: **HAMA Maoki**
**Himeji-shi, Hyogo 672-8076 (JP)**

(74) Representative: **Nederlandsch Octrooibureau**
**P.O. Box 29720**
**2502 LS The Hague (NL)**

(54) **ABSORPTION BODY AND ABSORPTIVE ARTICLE**

(57)     An absorber 10 contains water-absorbent resin particles 10a and a fibrous substance, a medium particle diameter of the water-absorbent resin particles 10a is 250 to 600 $\mu$m, the water-absorbent resin particles 10a contain small-diameter particles having a particle diameter of 180 $\mu$m or less, and a falloff rate of a mixture of the small-diameter particles and the fibrous substance when the mixture is subjected to shaking treatment for 10 minutes is 20% by mass or less.

Fig.1

**Description**

**Technical Field**

The present invention relates to an absorber and an absorbent article.

**Background Art**

In the related art, an absorber containing water-absorbent resin particles has been used in an absorbent article for absorbing urine, sweat, or the like having water as a main component. It is known that the absorber contains fibrous substances in addition to the water-absorbent resin particles (for example, refer to Patent Literature 1 below).

**Citation List**

**Patent Literature**

[Patent Literature 1] Japanese Unexamined Patent Publication No. 2010-185029

**Summary of Invention**

**Technical Problem**

When producing an absorber used for an absorbent article, there is a case where a mass of the absorber becomes smaller than a total amount of a raw material due to the raw material falling off during a production process and an yield decreases. Therefore, it is required that the absorber can be produced with a good yield.
An object of an aspect of the present invention is to provide an absorber that can be produced with a good yield. In addition, an object of another aspect of the present invention is to provide an absorbent article using the absorber.

**Solution to Problem**

The absorber can contain water-absorbent resin particles having various particle diameters. In this regard, the present inventor has found that, although absorbers containing water-absorbent resin particles having various particle diameters and fibrous substances have the same falloff rate in a case of performing a falloff test on the absorbers, when producing an absorber using the water-absorbent resin particles and the fibrous substances, there is a case where an yield of an absorber is inferior whereas an yield of another absorber is better. In addition, the present inventor has found that, when absorbers are produced using water-absorbent resin particles having the same content of small-diameter particles of the particle diameter of 180 $\mu$m or less (hereinafter, simply referred to as "small-diameter particle" depending on the case) as the content of particles having a small particle diameter, even in a case where the content of the small-diameter particles is small, there is a case where an yield of an absorber is inferior whereas an yield of another absorber is better. In response to these findings, the present inventor has found that, in a case where a falloff rate of a mixture of small-diameter particles and a fibrous substance is within a specific range when performing a falloff test on the mixture, an absorber can be produced with a good yield using water-absorbent resin particles containing small-diameter particles and a fibrous substance.
An aspect of the present invention provides an absorber containing water-absorbent resin particles and a fibrous substance, in which a medium particle diameter of the water-absorbent resin particles is 250 to 600 $\mu$m, the water-absorbent resin particles contain small-diameter particles having a particle diameter of 180 $\mu$m or less, and a falloff rate of a mixture of the small-diameter particles and the fibrous substance when the mixture is subjected to shaking treatment for 10 minutes is 20% by mass or less. This absorber can be produced with a good yield.
Another aspect of the present invention provides a water-absorbent article including the above-mentioned absorber.

**Advantageous Effects of Invention**

According to an aspect of the present invention, it is possible to provide an absorber that can be produced with a good yield. In addition, according to another aspect of the present invention, it is possible to provide an absorbent article using the absorber. According to another aspect of the present invention, it is possible to provide use of an absorber and an absorbent article to the absorption of a liquid.

**Brief Description of Drawings**

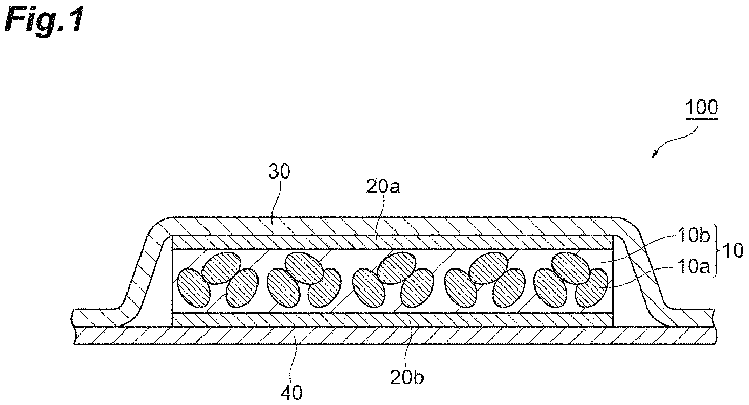

Fig. 1 is a cross-sectional view showing an example of an absorbent article.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacryl" are collectively referred to as "(meth)acrylic". Similarly, "acrylate" and "methacrylate" are also referred to as "(meth)acrylate". In a numerical value range described in stages in the present specification, an upper limit value or a lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in the examples. "Water-soluble" means that it exhibits a solubility in water of 5% by mass or more at 25°C. Materials exemplified in the present specification may be used alone, or may be used in combination of two or more. The content of each component in the composition means the total amount of a plurality of substances present in the composition in a case where the plurality of substances corresponding to each component are present in the composition, unless otherwise specified.

The absorber of the present embodiment contains water-absorbent resin particles and a fibrous substance. In the present embodiment, the medium particle diameter of the water-absorbent resin particles is 250 to 600 $\mu$m, and the water-absorbent resin particles contain small-diameter particles of the particle diameter of 180 $\mu$m or less (water-absorbent resin particles having the particle diameter of 180 $\mu$m or less), a falloff rate (hereinafter, referred to as "falloff rate D") of a mixture of the small-diameter particles and the fibrous substance when performing shaking treatment on the mixture for 10 minutes is 20% by mass or less. The absorber of the present embodiment can be used for absorption of a liquid such as urine, sweat, and blood (for example, menstrual blood).

According to the present embodiment, since the falloff rate D is in the above-mentioned range, the absorber can be produced with a good yield. According to the present embodiment, since the falloff rate D is in the above-mentioned range, the absorber can be produced with a good yield even in a case where the content of the small-diameter particles is small.

The falloff rate D is a falloff rate of a test piece when a test piece which is a mixture of small-diameter particles and a fibrous substance is prepared and the test piece is subjected to shaking treatment for 10 minutes. For example, the falloff rate D can be calculated by operation of "(1) a test piece is placed on a sieve having the opening of 850 $\mu$m; (2) the test piece is subjected to vibration for 5 minutes using a sieve shaker; (3) after exchanging the top and the bottom of the test piece, the test piece is subjected to vibration for 5 minutes in the same manner; (4) a falloff product of the water-absorbent resin and a pulverized pulp fallen off from the test piece is collected". The falloff rate D is a falloff rate of a dry test piece. The test piece contains a fibrous substance of the same type as that of the fibrous substance of the absorber of the present embodiment. The content of the fibrous substance in the test piece and the absorber may be the same, or may be different. For example, the test piece is made of small-diameter particles and a fibrous substance. The test piece may be obtained by mixing the same amount of small-diameter particles and the fibrous substance with each other.

The falloff rate D is 20% by mass or less from a viewpoint of producing an absorber with a good yield. The falloff rate D is preferably 18% by mass or less, more preferably 17% by mass or less, further more preferably 16% by mass or less, particularly preferably 15% by mass or less, extremely preferably 10% by mass or less, extraordinarily preferably 8% by mass or less, and even further more preferably 7% by mass or less, from a viewpoint of easily producing an absorber with a good yield. The lower limit of the falloff rate D may be 0% by mass or more, and may exceed 0% by mass.

The medium particle diameter of the water-absorbent resin particles is preferably 250 $\mu$m or more, more preferably 260 $\mu$m or more, further more preferably 280 $\mu$m or more, particularly preferably 300 $\mu$m or more, and extremely preferably 350 $\mu$m or more, from a viewpoint of good handling in producing an absorber and from a viewpoint of easily producing an absorber with a good yield. The medium particle diameter of the water-absorbent resin particles is preferably 600 $\mu$m or less, more preferably 580 $\mu$m or less, further more preferably 560 $\mu$m or less, particularly preferably 520 $\mu$m or less, extremely preferably 500 $\mu$m or less, and extraordinarily preferably 480 $\mu$m or less, from a viewpoint of easily producing an absorber with good touch feeling and from a viewpoint of easily producing an absorber with a good yield. From these viewpoints, the medium particle diameter of the water-absorbent resin particles is preferably 250 to 600 $\mu$m.

The content of the small-diameter particles having the particle diameter of 180 $\mu$m or less in the water-absorbent resin particles is preferably in the following range based on a total mass of the water-absorbent resin particles. The content of the small-diameter particles is preferably 15% by mass or less, more preferably 12% by mass or less, further more preferably 10% by mass or less, particularly preferably 8% by mass or less, and extremely preferably 7% by mass or less, from a viewpoint of good handleability at the time of producing the absorber. The content of the small-diameter particles is preferably 1% by mass or more, more preferably 2% by mass or more, and further more preferably 3% by mass or more, from a viewpoint of enhancing the productivity of the water-absorbent resin particles. From these viewpoints, the content of the small-diameter particles is preferably 1 to 15% by mass.

The water-absorbent resin particles may contain particles other than small-diameter particles having the particle diameter of 180 μm or less. Examples of the particles which can be contained in the water-absorbent resin particles include particles having the particle diameter of more than 180 μm and 300 μm or less (hereinafter, referred to as "particle A"), particles having the particle diameter of more than 300 μm and 400 μm or less (hereinafter, referred to as "particle B"), particles having the particle diameter of more than 400 μm and 500 μm or less (hereinafter, referred to as "particle C"), particles having the particle diameter of more than 500 μm and 850 μm or less (hereinafter, referred to as "particle D"), and particles having the particle diameter of more than 850 μm (hereinafter, referred to as "particle E").

The water-absorbent resin particles (containing small-diameter particles) can further contain additional components such as a gel stabilizer, a metal chelating agent (for example, diethylenetriamine pentasodium pentaacetate), and a flowablility improver (lubricant). Additional components can be disposed inside the polymer particles, on the surface of the polymer particles, or both thereof.

The water-absorbent resin particles (containing small-diameter particles) may contain a plurality of inorganic particles disposed on the surface of the polymer particles. For example, by mixing the polymer particles and the inorganic particles, it is possible to dispose the inorganic particles on the surface of the polymer particles. The inorganic particles may be silica particles such as amorphous silica.

In a case where the water-absorbent resin particles include inorganic particles disposed on the surface of the polymer particles, the content of the inorganic particles may be in the following range based on the total mass of the polymer particles. The content of the inorganic particles may be 0.05% by mass or more, 0.1% by mass or more, 0.15% by mass or more, or 0.2% by mass or more. The content of the inorganic particles may be 5.0% by mass or less, 3.0% by mass or less, 1.0% by mass or less, 0.5% by mass or less, or 0.3% by mass or less. In a case where the content of the inorganic particles is within the above range, water-absorbent resin particles having the suitable falloff rate of a mixture of the small-diameter particles and the fibrous substance when the mixture is subjected to a falloff test is easily obtained.

The inorganic particles here usually have a minute size as compared with the size of the polymer particles. For example, the average particle diameter of the inorganic particles may be 0.1 to 50 μm, 0.5 to 30 μm, or 1 to 20 μm. The average particle diameter can be measured by a dynamic light scattering method or a laser diffraction/scattering method.

The absorber of the present embodiment contains a fibrous substance, and for example, is a mixture containing water-absorbent resin particles and a fibrous substance. For example, the structure of the absorber may be a structure in which the water-absorbent resin particles and the fibrous substance are uniformly mixed, may be a structure in which the water-absorbent resin particles are sandwiched between the fibrous substances formed in the form of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester and polyolefin; and a mixture of these fibers. The fibrous substance may be used alone, or may be used in combination of two or more. As the fibrous substance, hydrophilic fibers, water-absorbent fibers, or the like can be used.

The content (mass ratio) of the water-absorbent resin particles in the absorber may be 2% by mass or more and less than 100% by mass, or may be 10% to 80% by mass or 20% to 60% by mass, with respect to the total of the water-absorbent resin particles and the fibrous substance.

The content of the water-absorbent resin particles in the absorber is preferably 100 to 1000 g, more preferably 150 to 800 g, and further more preferably 200 to 700 g per 1 m$^2$ of the absorber from a viewpoint of easily obtaining sufficient water absorption performance. The content of the fibrous substance in the absorber is preferably 50 to 800 g, more preferably 100 to 600 g, and further more preferably 150 to 500 g per 1 m$^2$ of the absorber from a viewpoint of easily obtaining sufficient water absorption performance.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more.

Examples of the thermal bonding synthetic fiber include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesive include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsion include a polymerization product of at least one monomer selected from the group consisting of methyl methacrylate, styrene, acrylonitrile, 2-ethylhexyl acrylate, butyl acrylate, butadiene, ethylene, and vinyl acetate.

The shape of the absorber of the present embodiment may be a sheet shape, for example. The thickness of the absorber (for example, thickness of the sheet shape of absorber) may be 0.1 to 20 mm or 0.3 to 15 mm, for example.

The water-absorbent resin particles used in the present embodiment may be any water-absorbent resin particles as long as the water-absorbent resin particles can retain water, and the liquid to be absorbed can contain water. The water-absorbent resin particles can have high water-absorbing ability with respect to physiological saline. The water retention amount of physiological saline of the water-absorbent resin particles is preferably 20 g/g or more, 25 g/g or more, 27 g/g or more, 30 g/g or more, 32 g/g or more, 35 g/g or more, 37 g/g or more, 39 g/g or more, or 40 g/g or more, from a viewpoint of easily suitably enhancing absorption capacity of the absorber. The water retention amount of physiological saline of the water-absorbent resin particles may be 70 g/g or less, 65 g/g or less, 60 g/g or less, 57 g/g or less, 55 g/g or less, 52 g/g or less, 50 g/g or less, 47 g/g or less, 45 g/g or less, or 43 g/g or less. The water retention amount of physiological saline of the water-absorbent resin particles may be 20 to 70 g/g, 25 to 65 g/g, 27 to 60 g/g, 30 to 57 g/g, or 32 to 55 g/g, for example. As the water retention amount of physiological saline of the water-absorbent resin particles, a water retention amount at 25°C can be used. The water retention amount of physiological saline of the water-absorbent resin particles can be measured by the method described in International Publication No. 2018/181565.

Examples of the shape of the water-absorbent resin particles include substantially spherical, crushed, and granular shapes, and crushed or granular shape is preferable from a viewpoint of easily producing an absorber with a good yield. The water-absorbent resin particles may have a desired particle size distribution at the time of being obtained by a production method to be described later, but the particle size distribution may be adjusted by performing an operation such as particle size adjustment using classification with a sieve.

The water-absorbent resin particles can contain a crosslinking polymer obtained by polymerizing a monomer containing an ethylenically unsaturated monomer (crosslinking polymer having a structural unit derived from the ethylenically unsaturated monomer), for example. That is, the water-absorbent resin particles can have a structural unit derived from an ethylenically unsaturated monomer. Examples of the polymerization method include a reverse phase suspension polymerization method, an aqueous solution polymerization method, a bulk polymerization method, and a precipitation polymerization method. Among these, the reverse phase suspension polymerization method or the aqueous solution polymerization method is preferable from a viewpoint of ensuring good characteristics of the obtained water-absorbent resin particles and facilitating control of the polymerization reaction. In the following, as a method for polymerizing an ethylenically unsaturated monomer, a reverse phase suspension polymerization method will be described as an example.

The ethylenically unsaturated monomer is preferably water-soluble, and examples thereof include (meth)acrylic acid and a salt thereof, 2-(meth)acrylamide-2-methylpropanesulfonic acid and a salt thereof, (meth)acrylamide, N, N-dimethyl (meth)acrylamide, 2-hydroxyethyl (meth)acrylate, N-methylol (meth)acrylamide, polyethylene glycol mono(meth)acrylate, N, N-diethylaminoethyl (meth)acrylate, N, N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone, or may be used in combination of two or more. Functional groups such as a carboxyl group and an amino group of the above-mentioned monomers can function as functional groups capable of crosslinking in a surface crosslinking step to be described later.

Among these, from a viewpoint of industrial availability, the ethylenically unsaturated monomer preferably contains at least one compound selected from the group consisting of (meth)acrylic acid and a salt thereof, acrylamide, methacrylamide, and N, N-dimethylacrylamide, and more preferably contains at least one compound selected from the group consisting of (meth)acrylic acid and a salt thereof, and acrylamide. From a viewpoint of further enhancing the water-absorbent characteristics, the ethylenically unsaturated monomer further more preferably contains at least one compound selected from the group consisting of (meth)acrylic acid and a salt thereof. That is, the water-absorbent resin particles preferably have a structural unit derived from at least one selected from the group consisting of (meth)acrylic acid and a salt thereof.

As the monomer for obtaining the water-absorbent resin particles, a monomer other than the above-mentioned ethylenically unsaturated monomer may be used. Such a monomer can be used by being mixed with an aqueous solution containing the above-mentioned ethylenically unsaturated monomer, for example. The use amount of the ethylenically unsaturated monomer is preferably 70 to 100 mol% with respect to a total amount of the monomer (the total amount of the monomer for obtaining the water-absorbent resin particles. For example, a total amount of the monomers that provide a structural unit of the crosslinking polymer. The same applies hereinafter). Among these, the ratio of (meth)acrylic acid and a salt thereof is more preferably 70 to 100 mol% with respect to the total amount of the monomers. "Ratio of (meth)acrylic acid and a salt thereof" means the ratio of the total amount of (meth)acrylic acid and a salt thereof.

According to the present embodiment, as an example of the water-absorbent resin particles, it is possible to provide water-absorbent resin particles containing a crosslinking polymer having a structural unit derived from the ethylenically unsaturated monomer, in which the ethylenically unsaturated monomer contains at least one compound selected from the group consisting of (meth)acrylic acid and a salt thereof, and the ratio of (meth)acrylic acid and a salt thereof is 70 to 100 mol% with respect to the total amount of the monomer for obtaining the water-absorbent resin particles (for example, the total amount of the monomer that provides a structural unit of the crosslinking polymer).

The ethylenically unsaturated monomer is usually preferably used as an aqueous solution. The concentration of the ethylenically unsaturated monomer in the aqueous solution containing the ethylenically unsaturated monomer (hereinafter, simply referred to as "monomer aqueous solution") is preferably 20% by mass or more and a saturated concentration or less, more preferably 25 to 70% by mass, and further more preferably 30 to 55% by mass. Examples of the water used in the aqueous solution include tap water, distilled water, and ion-exchanged water.

In a case where the ethylenically unsaturated monomer has an acid group, the monomer aqueous solution may be used by neutralizing the acid group with an alkaline neutralizing agent. The degree of neutralization of the ethylenically unsaturated monomer by the alkaline neutralizing agent is preferably 10 to 100 mol%, more preferably 50 to 90 mol%, and further more preferably 60 to 80 mol% of the acid group in the ethylenically unsaturated monomer, from a viewpoint of increasing an osmotic pressure of the obtained water-absorbent resin particles, and further increasing the water-absorbent characteristics (water retention amount and the like). Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or may be used in combination of two or more. The alkaline neutralizing agent may be used in the form of an aqueous solution to simplify the neutralization operation. Neutralization of the acid group of the ethylenically unsaturated monomer can be performed by adding an aqueous solution of sodium hydroxide, potassium hydroxide, or the like dropwise in the above-mentioned monomer aqueous solution and mixing therewith.

In a reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like. As the radical polymerization initiator, a water-soluble radical polymerization initiator can be used.

Examples of the surfactant include a nonionic surfactant, and an anionic surfactant. Examples of the nonionic surfactant include sorbitan fatty acid ester, (poly)glycerin fatty acid ester ("(poly)" means both of a case where there is a prefix of "poly" and a case where there is no prefix thereof. The same applies hereinafter), sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester. Examples of the anionic surfactant include fatty acid salt, alkylbenzene sulfonate, alkylmethyl taurate, polyoxyethylene alkylphenyl ether sulfate, polyoxyethylene alkyl ether sulfonate, phosphate ester of polyoxyethylene alkyl ether, and phosphate ester of polyoxyethylene alkylallyl ether. The surfactant may be used alone, or may be used in combination of two or more.

From a viewpoint of a good state of the W/O type reverse phase suspension, easily obtaining water-absorbent resin particles having a suitable particle diameter, and industrial availability, the surfactant preferably contains at least one compound selected from the group consisting of a sorbitan fatty acid ester, a polyglycerin fatty acid ester and a sucrose fatty acid ester. From a viewpoint of easily producing an absorber with a good yield in a case of using the obtained water-absorbent resin particles, the surfactant preferably contains at least one selected from the group consisting of sorbitan fatty acid ester and sucrose fatty acid ester, and more preferably contains at least one selected from the group consisting of sorbitan monolaurate and sucrose stearic acid ester.

The use amount of the surfactant is preferably 0.05 to 10 parts by mass, more preferably 0.08 to 5 parts by mass, and further more preferably 0.1 to 3 parts by mass with respect to 100 parts by mass of the monomer aqueous solution from a viewpoint of obtaining a sufficient effect on the use amount and economic efficiency.

In the reverse phase suspension polymerization, a polymeric dispersant may be used in combination with the above-mentioned surfactant. Examples of the polymeric dispersant include maleic anhydride-modified polyethylene, maleic anhydride-modified polypropylene, maleic anhydride-modified ethylene/propylene copolymer, maleic anhydride-modified EPDM (ethylene propylene diene terpolymer), maleic anhydride-modified polybutadiene, maleic anhydride/ethylene copolymer, maleic anhydride/propylene copolymer, maleic anhydride/ethylene/propylene copolymer, maleic anhydride/butadiene copolymer, polyethylene, polypropylene, ethylene/propylene copolymer, oxidized polyethylene, oxidized polypropylene, oxidized ethylene/propylene copolymer, ethylene/acrylic acid copolymer, ethyl cellulose, and ethyl hydroxyethyl cellulose. The polymeric dispersant may be used alone or may be used in combination of two or more. From a viewpoint of better dispersion stability of the monomer, the polymeric dispersant is preferably at least one selected from the group consisting of maleic anhydride-modified polyethylene, maleic anhydride-modified polypropylene, maleic anhydride-modified ethylene/propylene copolymer, maleic anhydride/ethylene copolymer, maleic anhydride/propylene copolymer, maleic anhydride/ethylene/propylene copolymer, polyethylene, polypropylene, ethylene/propylene copolymer, oxidized polyethylene, oxidized polypropylene, and oxidized ethylene/propylene copolymer.

The use amount of the polymeric dispersant is preferably 0.05 to 10 parts by mass, more preferably 0.08 to 5 parts by mass, and further more preferably 0.1 to 3 parts by mass with respect to 100 parts by mass of the monomer aqueous solution, from a viewpoint of obtaining a sufficient effect on the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or may be used in combination of two or more.

The hydrocarbon dispersion medium may contain at least one selected from the group consisting of n-heptane and cyclohexane from a viewpoint of industrial availability and stable quality. In addition, from the same viewpoint, as the mixture of the above-mentioned hydrocarbon dispersion medium, for example, commercially available Exxsol Heptane (manufactured by ExxonMobil: containing 75% to 85% of n-heptane and isomeric hydrocarbons) may be used.

The use amount of the hydrocarbon dispersion medium is preferably 30 to 1000 parts by mass, more preferably 40 to 500 parts by mass, and further more preferably 50 to 300 parts by mass with respect to 100 parts by mass of the monomer aqueous solution, from a viewpoint of appropriately removing the heat of polymerization and easily controlling the polymerization temperature. In a case where the use amount of the hydrocarbon dispersion medium is 30 parts by mass or more, the polymerization temperature tends to be easily controlled. In a case where the use amount of the hydrocarbon dispersion medium is 1000 parts by mass or less, the productivity of polymerization tends to be improved, which is economical.

The radical polymerization initiator is preferably water-soluble, and examples thereof include persulfates such as potassium persulfate, ammonium persulfate, and sodium persulfate; peroxides such as methyl ethyl ketone peroxide, methyl isobutyl ketone peroxide, di-t-butyl peroxide, t-butyl cumylperoxide, t-butylperoxyacetate, t-butylperoxyisobutyrate, t-butylperoxypivalate, and hydrogen peroxide; azo compounds such as 2,2'-azobis(2-amidinopropane) dihydrochloride, 2,2'-azobis [2-(N-phenylamidino) propane] dihydrochloride, 2,2'-azobis [2-(N-allylamidino) propane] dihydrochloride, 2,2'-azobis [2-(2-imidazoline-2-yl) propane] dihydrochloride, 2,2'-azobis {2-[1-(2-hydroxyethyl) -2-imidazoline-2-yl] propane} dihydrochloride, 2,2'-azobis {2-methyl-N-[1,1-bis(hydroxymethyl)-2-hydroxyethyl]propionamide}, 2,2'-azobis [2-methyl-N-(2-hydroxyethyl)-propionamide], and 4,4'-azobis (4-cyanovaleric acid). The radical polymerization initiator may be used alone, or may be used in combination of two or more. The radical polymerization initiator is preferably at least one selected from the group consisting of potassium persulfate, ammonium persulfate, sodium persulfate, 2,2'-azobis (2-amidinopropane) dihydrochloride, 2,2'-azobis [2-(2-imidazoline-2-yl) propane] dihydrochloride, and 2,2'-azobis {2-[1-(2-hydroxyethyl)-2-imidazoline-2-yl]propane}dihydrochloride.

The use amount of the radical polymerization initiator may be 0.00005 to 0.01 mol with respect to 1 mol of the ethylenically unsaturated monomer. In a case where the use amount of the radical polymerization initiator is 0.00005 mol or more, the polymerization reaction does not require a long time and is efficient. In a case where the use amount of the radical polymerization initiator is 0.01 mol or less, the occurrence of a rapid polymerization reaction is easily inhibited.

The above-mentioned radical polymerization initiator can also be used as a redox polymerization initiator in combination with a reducing agent such as sodium sulfite, sodium hydrogen sulfite, ferrous sulfate, and L-ascorbic acid.

At the time of the polymerization reaction, the monomer aqueous solution used for the polymerization may contain a chain transfer agent. Examples of the chain transfer agent include hypophosphites, thiols, thiolic acids, secondary alcohols, and amines.

The monomer aqueous solution used for the polymerization may contain a thickener in order to control the particle diameter of the water-absorbent resin particles. Examples of the thickener include hydroxyethyl cellulose, hydroxypropyl cellulose, methyl cellulose, carboxymethyl cellulose, polyethylene glycol, polyacrylamide, polyethyleneimine, dextrin, sodium alginate, polyvinyl alcohol, polyvinylpyrrolidone, and polyethylene oxide. In a case where the stirring speed at the time of polymerization is the same, the higher the viscosity of the monomer aqueous solution, the larger the medium particle diameter of the obtained particles tends to be.

Crosslinking by self-crosslinking may occur during polymerization, but crosslinking may be further performed by using an internal crosslinking agent. In a case where an internal crosslinking agent is used, the characteristics of the water-absorbent resin particles are easily controlled. The internal crosslinking agent is usually added to a reaction solution during the polymerization reaction. Examples of the internal crosslinking agent include di or tri (meth)acrylic acid esters of polyols such as ethylene glycol, propylene glycol, trimethylolpropane, glycerin, polyoxyethylene glycol, polyoxypropylene glycol, polyglycerin, and polyethylene glycol di(meth)acrylate; unsaturated polyesters obtained by reacting the above-mentioned polyols with unsaturated acids (such as maleic acid and fumaric acid); bis (meth)acrylamides such as N, N'-methylene bis (meth)acrylamide; di or tri (meth)acrylic acid esters obtained by reacting polyepoxide with (meth)acrylic acid; di (meth)acrylic acid carbamil esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N, N', N''-triallyl isocyanurate, and divinylbenzene; polyglycidyl compounds such as (poly)ethylene glycol diglycidyl ether, (poly) propylene glycol diglycidyl ether, (poly)glycerin diglycidyl ether, (poly)glycerin triglycidyl ether, (poly)propylene glycol polyglycidyl ether, and polyglycerol polyglycidyl ether; haloepoxy compounds such as epichlorohydrin, epibromhydrin, and $\alpha$-methylepichlorohydrin; and compounds having two or more reactive functional groups such as isocyanate compounds (2,4-tolylene diisocyanate and hexamethylene diisocyanate). The internal crosslinking agent may be used alone, or may be used in combination of two or more. The internal crosslinking agent is preferably a polyglycidyl compound, more preferably a diglycidyl ether compound, and further more preferably at least one selected from the group consisting of (poly)ethylene glycol diglycidyl ether, (poly)propylene glycol diglycidyl ether, and (poly)glycerin diglycidyl ether.

The use amount of the internal crosslinking agent is preferably 0 to 0.03 mol, more preferably 0.00001 to 0.01 mol, and further more preferably 0.00002 to 0.005 mol per 1 mol of the ethylenically unsaturated monomer from a viewpoint of suppressing water-soluble property by appropriately crosslinking the obtained polymer to easily obtain the sufficient water absorption amount.

It is possible to perform heating while stirring in a state of mixing an ethylenically unsaturated monomer, a radical polymerization initiator, a surfactant, a polymeric dispersant, a hydrocarbon dispersion medium, or the like (if necessary, additionally an internal crosslinking agent), and to perform reverse phase suspension polymerization in a water-in-oil system.

When performing the reverse phase suspension polymerization, a monomer aqueous solution containing an ethylenically unsaturated monomer is dispersed in a hydrocarbon dispersion medium in the presence of a surfactant (if necessary, additionally a polymeric dispersant). At this time, before the start of the polymerization reaction, the timing of adding the surfactant, the polymeric dispersant, or the like may be either before or after the addition of the monomer aqueous solution.

Among these, from a viewpoint of easily reducing the amount of the hydrocarbon dispersion medium remaining in the obtained water-absorbent resin, it is preferable to perform polymerization after dispersing the monomer aqueous solution in the hydrocarbon dispersion medium in which the polymeric dispersant is dispersed and then further dispersing the surfactant.

Reverse phase suspension polymerization can be performed in one stage, or in multiple stages of two or more stages. Reverse phase suspension polymerization is preferably performed in two to three stages from a viewpoint of increasing productivity.

In a case where reverse phase suspension polymerization is performed in multiple stages of two or more stages, a first stage reverse phase suspension polymerization is performed, an ethylenically unsaturated monomer is added to the reaction mixture obtained in the first polymerization reaction and mixed therewith, and second and subsequent stages of reverse phase suspension polymerization may be performed in the same method as the first stage. In the reverse phase suspension polymerization in each stage of the second and subsequent stages, in addition to the ethylenically unsaturated monomer, the above-mentioned radical polymerization initiator and/or internal crosslinking agent is preferably added in a range of a molar ratio of each component with respect to the above-mentioned ethylenically unsaturated monomer, based on an amount of the ethylenically unsaturated monomer added at the time of the second and subsequent stages of reverse phase suspension polymerization, to perform reverse phase suspension polymerization. In the reverse phase suspension polymerization in each stage of second and subsequent stages, an internal crosslinking agent may be used if necessary. In a case of using the internal crosslinking agent, the internal crosslinking agent is preferably added within a range of the molar ratio of each component with respect to the above-mentioned ethylenically unsaturated monomer based on the amount of the ethylenically unsaturated monomer provided in each stage, to perform reverse phase suspension polymerization.

The temperature of the polymerization reaction varies depending on the used radical polymerization initiator, and the temperature is preferably 20°C to 150°C, and more preferably 40°C to 120°C from a viewpoint of rapidly proceeding the polymerization and shortening the polymerization time to enhance economic efficiency, and easily removing polymerization heat and smoothly performing reaction. The reaction time is usually 0.5 to 4 hours. The completion of the polymerization reaction can be confirmed by stopping the temperature rise in the reaction system. Thus, the polymer of the ethylenically unsaturated monomer is usually obtained in a state of a hydrogel.

After the polymerization, a crosslinking agent may be added to the obtained hydrogel-like polymer and heated to perform post-polymerization crosslinking. By performing post-polymerization crosslinking, a degree of crosslinking of the hydrogel-like polymer can be increased, and the characteristics can be further improved.

Examples of the crosslinking agent for performing post-polymerization crosslinking include polyols such as ethylene glycol, propylene glycol, 1,4-butanediol, trimethylolpropane, glycerin, polyoxyethylene glycol, polyoxypropylene glycol, and polyglycerin; compounds having two or more epoxy groups such as (poly)ethylene glycol diglycidyl ether, (poly)propylene glycol diglycidyl ether, and (poly)glycerin diglycidyl ether; haloepoxy compounds such as epichlorohydrin, epibromhydrin, and α-methylepichlorohydrin; compounds having two or more isocyanate groups such as 2,4-tolylene diisocyanate and hexamethylene diisocyanate; oxazoline compounds such as 1,2-ethylenebisoxazoline; carbonate compounds such as ethylene carbonate; and hydroxyalkylamide compounds such as bis [N, N-di (β-hydroxyethyl)] adipamide. Among these, polyglycidyl compounds such as (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether are preferable. The crosslinking agent may be used alone, or may be used in combination of two or more.

The amount of the crosslinking agent used for post-polymerization crosslinking is preferably 0 to 0.03 mol, more preferably 0 to 0.01 mol, and further more preferably 0.00001 to 0.005 mol per 1 mol of a water-soluble ethylenically unsaturated monomer from a viewpoint of exhibiting suitable characteristics by appropriately crosslinking the obtained hydrogel-like polymer.

The timing of adding the crosslinking agent used for post-polymerization crosslinking may be after the polymerization of the ethylenically unsaturated monomer used for the polymerization, and in the case of multiple-stage polymerization, it is preferable to add the crosslinking agent after the multiple-stage polymerization. Considering fluctuation in water due to heat generation at the time of polymerization and after polymerization, retention due to process delay, opening of the system at the time of addition of the crosslinking agent, addition of water due to the addition of the crosslinking agent, or the like, the crosslinking agent for post-polymerization crosslinking is preferably added in a region of [water content (immediately after polymerization) ± 3% by mass] from a viewpoint of water content (to be described later).

Subsequently, the polymer particles (for example, polymer particles having a structural unit derived from an ethylenically unsaturated monomer) are obtained by drying in order to remove water from the obtained hydrogel-like polymer. Examples of a drying method include (a) a method of removing water by performing azeotropic distillation by heating from outside in a state where a hydrogel-like polymer is dispersed in a hydrocarbon dispersion medium, and refluxing the hydrocarbon dispersion medium, (b) a method of taking out a hydrogel-like polymer by decantation and drying under reduced pressure, and (c) a method of filtering the hydrogel-like polymer with a filter and drying under reduced pressure. Among these, it is preferable to use the method (a) due to the simplicity in the production process.

It is possible to adjust the particle diameter of water-absorbent resin particles by adjusting a rotation speed of a stirrer during the polymerization reaction, or by adding a flocculant into the system after the polymerization reaction or in the initial stage of drying. By adding a flocculant, it is possible to increase the particle diameter of the obtained water-absorbent resin particles. As the flocculant, an inorganic flocculant can be used. Examples of the inorganic flocculant (for example, powdered inorganic flocculant) include silica, zeolite, bentonite, aluminum oxide, talc, titanium dioxide, kaolin, clay, and hydrotalcite. From a viewpoint of better flocculation effect, the flocculant is preferably at least one selected from the group consisting of silica, aluminum oxide, talc, and kaolin.

In the reverse phase suspension polymerization, a method of adding the flocculant is preferably a method of preliminarily dispersing a flocculant in a hydrocarbon dispersion medium or water of the same type as that used in the polymerization, and then mixing into a hydrocarbon dispersion medium containing a hydrogel-like polymer under stirring.

The addition amount of the flocculant is preferably 0.001 to 1 part by mass, more preferably 0.005 to 0.5 part by mass, and further more preferably 0.01 to 0.2 parts by mass with respect to 100 parts by mass of the ethylenically unsaturated monomer used for the polymerization. In a case where the addition amount of the flocculant is within the above-mentioned range, water-absorbent resin particles having a target particle size distribution can be easily obtained.

In the production of the water-absorbent resin particles, it is preferable to perform crosslinking (surface crosslinking) of a surface portion of a hydrogel-like polymer using a crosslinking agent in a drying step or any subsequent steps. By performing surface crosslinking, the characteristics of the water-absorbent resin particles is easily controlled. The surface crosslinking is preferably performed at the timing when the hydrogel-like polymer has a specific water content. The timing of surface crosslinking is preferably when the water content of the hydrogel-like polymer is 5% to 50% by mass, more preferably when the water content of the hydrogel-like polymer is 10% to 40% by mass, and further more preferably when the water content of the hydrogel-like polymer is 15% to 35% by mass. The water content (mass%) of the hydrogel-like polymer is calculated by the following formula.

$$\text{Water content} = [\text{Ww}/(\text{Ww} + \text{Ws})] \times 100$$

Ww: Water amount of a hydrogel-like polymer obtained by adding water amount used if necessary when mixing a flocculant, a surface crosslinking agent, or the like to an amount obtained by subtracting water amount discharged to the outside of the system in the drying step, from water amount contained in a monomer aqueous solution before polymerization in the entire polymerization step.

Ws: Solid content calculated from the charged amount of materials such as ethylenically unsaturated monomer, crosslinking agent, and initiator that constitute a hydrogel-like polymer.

Examples of the crosslinking agent (surface crosslinking agent) for performing surface crosslinking include compounds having two or more reactive functional groups. Examples of the crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylpropane triglycidyl ether (poly)propylene glycol polyglycidyl ether, and (poly)glycerol polyglycidyl ether; haloepoxy compounds such as epichlorohydrin, epibromhydrin, and $\alpha$-methylepichlorohydrin; isocyanate compounds such as 2,4-tolylene diisocyanate and hexamethylenediisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; and hydroxyalkylamide compounds such as bis [N, N-di ($\beta$-hydroxyethyl)] adipamide. The crosslinking agent may be used alone, or may be used in combination of two or more. The crosslinking agent is preferably a polyglycidyl compound, and more preferably at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent is usually preferably 0.00001 to 0.02 mol, more preferably 0.00005 to 0.01 mol, and further more preferably 0.0001 to 0.005 mol with respect to 1 mol of the ethylenically unsaturated monomer used for polymerization from a viewpoint of easily obtaining suitable water-absorbent characteristics (water retention amount and the like). In addition, in a case where the use amount of the surface crosslinking agent is within the above range, water-absorbent resin particles having a suitable falloff rate of a mixture of small-diameter particles and a fibrous substance when performing falloff test on the mixture are easily obtained.

After surface crosslinking, it is possible to obtain polymer particles which are surface-crosslinked dried products by distilling water and a hydrocarbon dispersion medium with a known method.

Hereinafter, the aqueous solution polymerization method will be briefly described as another method for polymerizing the ethylenically unsaturated monomer. Examples of the polymerization method adopted when performing the aqueous solution polymerization method include static polymerization in which polymerization is performed in a state without stirring the monomer aqueous solution (for example, in a static state), and stirring polymerization in which polymerization is performed while stirring the monomer aqueous solution in a reaction device. As the constituents such as the ethylenically unsaturated monomer, the radical polymerization initiator, and the internal crosslinking agent, the same components as those in the reverse phase suspension polymerization method described above are used.

The polymerization method may be batch polymerization, semi-continuous polymerization, continuous polymerization (for example, continuous belt polymerization), or the like. For example, in the continuous polymerization of static polymerization in the aqueous solution polymerization method, it is possible to perform polymerization reaction while continuously supplying a monomer aqueous solution to a continuous polymerization device, and to obtain a continuous (for example, strip-shaped) hydrogel.

The aqueous solution polymerization method may include a rough crushing step of pulverizing a hydrogel-like polymer to obtain a roughly crushed product of the hydrogel-like polymer. In the rough crushing step, if necessary, temperature adjustment is performed on the hydrogel-like polymer, and the hydrogel-like polymer is subjected to the rough crushing treatment. As the rough crushing device for the hydrogel, a rough crushing device such as a kneader (pressurized kneader, double-armed kneader, and the like), a meat chopper, a cutter mill, and a pharma mill can be used. Among these, a double-armed kneader, a meat chopper, or a cutter mill is preferable. The rough crushing device may be the same type as the pulverizing device for the following hydrogel-like polymer dried product.

The aqueous solution polymerization method preferably includes a drying step of drying the hydrogel-like polymer roughly crushed product obtained in the rough crushing step to obtain a hydrogel-like polymer dried product. As the drying method, a solvent may be removed from the hydrogel-like polymer roughly crushed product by a general method such as natural drying, heat drying, spray drying, and freeze drying, and these methods may be used in combination. The drying temperature in a case where the drying is performed at normal pressure is preferably 70°C to 250°C. Drying is performed until the water content of the hydrogel-like polymer roughly crushed product becomes 10% by mass or less, for example.

The aqueous solution polymerization method preferably includes a pulverization step of pulverizing the hydrogel-like polymer dried product obtained in the drying step to obtain a pulverized product. In addition, the method for producing water-absorbent resin particles may include a classification step of classifying the pulverized product obtained in the pulverization step. The classified particles may be pulverized again, and the pulverization step and the classification step may be repeated.

A known pulverizer can be used for pulverizing the hydrogel-like polymer dried product. For example, a roller mill (roll mill), a stamp mill, a jet mill, a high-speed rotary crusher (hammer mill, pin mill, rotor beater mill, and the like), a container-driven mill (rotary mill, vibration mill, planetary mill, and the like), or the like can be used.

A known classification method can be used for classifying the pulverized product. For example, screen classification, wind power classification, or the like may be used, and screen classification is preferable. Screen classification is a method of classifying particles on a screen into particles that pass through a mesh of the screen and particles that do not pass through the screen by vibrating the screen. Wind power classification is a method of classifying particles using the flow of air.

The absorber of the present embodiment may contain an inorganic powder (for example, amorphous silica), a deodorant, an antibacterial agent, a fragrance, or the like. In a case where the water-absorbent resin particles contain inorganic particles, the absorber may contain an inorganic powder in addition to the inorganic particles of the water-absorbent resin particles.

The absorbent article of the present embodiment includes an absorber of the present embodiment. Examples of the absorbent article of the present embodiment include a core wrap that retains an absorber; a liquid permeable sheet disposed on the outermost part at the side where the liquid to be absorbed enters; and a liquid impermeable sheet disposed on the outermost part at the opposite side to the side where the liquid to be absorbed enters. Examples of the absorbent article include diapers (for example, paper diapers), toilet training pants, incontinence pads, sanitary materials (sanitary napkins, tampons, and the like), sweat pads, pet sheets, portable toilet members, and animal excrement treatment materials.

Fig. 1 is a cross-sectional view showing an example of an absorbent article. An absorbent article 100 shown in Fig. 1 includes an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has a water-absorbent resin particle 10a and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (lower side of the absorber 10 in Fig. 1) in a state of being in contact with the absorber 10. The absorber 10 is disposed between the core wrap 20a and the core wrap 20b. Examples of the core wraps 20a and 20b include tissues and non-woven fabrics. The core wrap 20a and the core wrap 20b have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on a lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited, and is appropriately adjusted according to the use of the absorbent article or the like. In addition, the method of retaining the shape of the absorber 10 using the core wraps 20a and 20b is not particularly limited, and as shown in Fig. 1, the absorber may be wrapped by a plurality of core wraps, and the absorber is wrapped by one core wrap.

According to the present embodiment, it is possible to provide a liquid absorbing method using the absorber or the absorbent article of the present embodiment. The liquid absorbing method of the present embodiment includes a step of bringing the liquid to be absorbed into contact with the absorber or the absorbent article of the present embodiment.

**Examples**

Hereinafter, contents of the present invention will be described in further detail using examples and comparative examples, but the present invention is not limited to the following examples.

<Preparation of water-absorbent resin particles>

(Example 1)

A round-bottomed cylindrical separable flask having 4 side wall baffles (baffle width: 7 mm), with the inner diameter of 11 cm and the internal volume of 2 L, equipped with a reflux cooling device, a dropping funnel, a nitrogen gas introduction tube, and a stirrer (a stirrer blade having two stages of four inclined paddle blades with the blade diameter of 5 cm (those surface-treated with fluororesin)) was prepared. Into this flask, 451.4 g of n-heptane as a hydrocarbon dispersion medium was put, and 1.288 g of sorbitan monolaurate (Nonion LP-20R, HLB value: 8.6, manufactured by NOF CORPORATION) was added as a surfactant to obtain a mixture. The sorbitan monolaurate was dissolved in n-heptane by raising the temperature of this mixture to 50°C while stirring at the rotation speed of 300 rpm of the stirrer, and then the internal temperature was cooled to 40°C.

92.0 g of 80.5% by mass aqueous acrylic acid solution (acrylic acid: 1.03 mol) was put into a triangular flask having the internal volume of 500 mL. Subsequently, while cooling with ice from the outside, 147.7 g of 20.9% by mass sodium hydroxide aqueous solution was added dropwise to neutralize the acrylic acid. Subsequently, by adding 0.101 g (0.373 mmol) of 2,2'-azobis (2-amidinopropane) dihydrochloride as a water-soluble radical polymerization initiator to the obtained acrylic acid partial neutralization aqueous solution, and then dissolving therein, a monomer aqueous solution was prepared.

After adding the above-mentioned monomer aqueous solution into the above-mentioned separable flask, the inside of the system was sufficiently replaced with nitrogen. Thereafter, while stirring at the rotation speed of 700 rpm of the stirrer, the flask was immersed in a water bath at 70°C and held for 60 minutes to complete the polymerization, to obtain a hydrogel-like polymer.

Thereafter, while stirring at the stirring speed of 1000 rpm of the stirrer, a dispersion obtained by previously dispersing 0.092 g of amorphous silica (Oriental Silicas Corporation, Tokusil NP-S) as a powdered inorganic flocculant to 100 g of n-heptane was added to a polymer solution containing the produced hydrogel-like polymer, n-heptane, and a surfactant, and then mixing was performed for 10 minutes. Thereafter, the flask containing the reaction solution was immersed in an oil bath at 125°C, and 106.5 g of water was extracted to the outside of the system while refluxing n-heptane by azeotropic distillation of n-heptane and water. Thereafter, 4.14 g of 2% by mass ethylene glycol diglycidyl ether aqueous solution (ethylene glycol diglycidyl ether: 0.475 mmol) was added as a surface crosslinking agent, and then the internal temperature was held at 83 ± 2°C for 2 hours.

Thereafter, water and n-heptane were evaporated at 120°C and dried until almost no evaporated product from the system was distilled off, to obtain a dried product. This dried product was passed through a sieve having the opening of 850 μm to obtain 90.5 g of water-absorbent resin particles.

(Example 2)

A 2 L of separable flask was charged with 141.2 g of 100% acrylic acid. After adding 95.9 g of ion-exchanged water while stirring in the separable flask, 261.2 g of 30% by mass sodium hydroxide was added dropwise under an ice bath. In addition, 75.9 g of 100% acrylic acid was added while stirring to prepare an acrylic acid partial neutralization solution having the monomer concentration of 45% by mass.

In a stainless steel container (diameter: 188 mm, height: 60 mm) equipped with a stirrer (diameter 8 mm, length 40 mm), 574.2 g of the acrylic acid partial neutralization solution as monomers used in polymerization, 163.5 g of ion-exchanged water, and 0.648 g of polyethylene glycol diacrylate (internal crosslinking agent, average number of polyethylene oxide units: 9) were added, the stirrer was then rotated to uniformly disperse, and thereby a mixture was obtained. After replacing the inside of the stainless steel container with nitrogen, the temperature of the mixture was adjusted to 18°C. Subsequently, 1.190 g of 5% by mass sodium persulfate aqueous solution, 1.190 g of 5% by mass 2,2'-azobis (2-amidinopropane) dihydrochloride aqueous solution, 1.12 g of 0.5% by mass L-ascorbic acid aqueous solution, and 1.26 g of 0.35% by mass hydrochloric acid aqueous solution were sequentially added dropwise under stirring at 400 rpm. The polymerization started immediately after the hydrogen peroxide was added dropwise, and the temperature reached 82°C 18 minutes after the start of the polymerization. After 20 minutes from the start of the polymerization, the obtained hydrogel-like polymer was immersed in a water bath at 80°C while being in a container and aged for 10 minutes, and then cooled at room temperature until the temperature became 40°C or lower.

The above-mentioned hydrogel-like polymer was cut into three parts. After charging one of divided products (about 250 g) into a double-armed kneader having the volume of 1 L, the hydrogel-like polymer was crushed at 29 rpm for 15 minutes. Subsequently, drying with hot air was performed at 180°C for 30 minutes to obtain a dried product. This dried product was pulverized under a condition of a trapezoidal hole of 1 mm on the screen using a pulverizer (Retsch, Rotor Beater Mill SR300). The pulverized particles were passed through a sieve having the opening of 850 μm, and the particles remaining on the sieve were pulverized again by the pulverizer. By repeating this operation, 130 g of particles having the particle diameter of less than 850 μm were obtained.

By classifying the pulverized particles with a sieve having the opening of 850 μm and a sieve having the opening of 180 μm, water-absorbent resin particles A2 which were the fraction that passed through the sieve having the opening of 850 μm and remained on the sieve having the opening of 180 μm, and water-absorbent resin particles B2 which were the fraction that passed through the sieve having the opening of 180 μm were obtained. In addition, a water-absorbent resin precursor was obtained by remixing a part of the water-absorbent resin particles B2 with the water-absorbent resin particles A2 so that the particle size distribution was close to the normal distribution.

30 g of the water-absorbent resin precursor was weighed in a round-bottomed cylindrical separable flask having the inner diameter of 11 cm equipped with an anchor-type stirrer blade made of fluororesin. Subsequently, while stirring at 300 rpm, a crosslinking aqueous solution obtained by mixing 0.015 g of ethylene glycol diglycidyl ether, 0.9 g of water, 0.300 g of propylene glycol, and 0.300 g of isopropyl alcohol was sprayed. The mixture obtained by this spraying was uniformly spread on a fluororesin-coated tray and then heated at 180°C for 40 minutes. After cooling to room temperature, water-absorbent resin particles were obtained by passing through a mesh of 850 $\mu$m.

(Example 3)

A 2 L separable flask was charged with 74.1 g of 100% acrylic acid. After adding 53.4 g of ion-exchanged water while stirring the inside of the separable flask, 104.8 g of 30% by mass sodium hydroxide was added dropwise under an ice bath to prepare an acrylic acid partial neutralization solution having the monomer concentration of 39% by mass.

To a stainless steel container (diameter: 135 mm, height: 145 mm) equipped with a stirrer (diameter 8 mm, length 40 mm), 232.3 g of the acrylic acid partial neutralization solution as a monomer used in polymerization, 5.52 g of 1% by mass ethylene glycol diglycidyl ether aqueous solution (internal crosslinking agent), 0.920 g of 2% by mass potassium persulfate aqueous solution (radical polymerization initiator), and 1.84 g of 5% by mass 2,2'-azobis (2-amidinopropane) dihydrochloride aqueous solution were added, the stirrer was then rotated to uniformly disperse, and thereby a mixture was obtained. While stirring the mixture in the stainless steel container at 400 rpm, the stainless steel container was immersed in a water bath at 60°C, and after 5 minutes, nitrogen was blown into the mixture. Heat generation, which seems to be the start of polymerization, was observed 9 minutes after the start of blowing nitrogen, and the temperature reached 104°C after 14 minutes. The obtained hydrogel-like polymer was immersed in a water bath at 75°C while being in a container and aged for 20 minutes, and then cooled at room temperature until the temperature became 40°C or lower.

The above-mentioned hydrogel-like polymer was cut into the size of 5 to 10 mm, and then dried with hot air at 180°C for 30 minutes to obtain a dried product. 10 g of the obtained dried product was collected, pulverized for 2 seconds with a small pulverizer (Wonder Blender WB-1), passed through a sieve having the opening of 850 $\mu$m, and the particles remaining on the sieve and under the sieve were separately recovered. The operation was performed on the total amount of the obtained dried product. After the pulverization of the total amount of the dried product was completed, the collected portion on the sieve was repeatedly pulverized and classified in the same manner as in the above operation to obtain 80 g of particles having the particle diameter of less than 850 $\mu$m.

By classifying the pulverized particles with a sieve having the opening of 850 $\mu$m and a sieve having the opening of 180 $\mu$m, the water-absorbent resin particles A3 which were the fraction that passed through the sieve having the opening of 850 $\mu$m and remained on the sieve having the opening of 180 $\mu$m and the water-absorbent resin particles B3 which were the fraction that passed through the sieve having the opening of 180 $\mu$m were obtained. In addition, a water-absorbent resin precursor was obtained by remixing a part of the water-absorbent resin particles B3 with the water-absorbent resin particles A3 so that the particle size distribution was close to the normal distribution.

30 g of the water-absorbent resin precursor was weighed in a round-bottomed cylindrical separable flask having the inner diameter of 11 cm equipped with an anchor-type stirrer blade made of fluororesin. Subsequently, while stirring at 300 rpm, a crosslinking aqueous solution obtained by mixing 0.060 g of ethylene glycol diglycidyl ether, 3.6 g of water, 1.20 g of propylene glycol, and 1.20 g of isopropyl alcohol was sprayed. The mixture obtained by this spraying was uniformly spread on a fluororesin-coated tray and then heated at 180°C for 40 minutes. After cooling to room temperature, water-absorbent resin particles were obtained by passing through a mesh of 850 $\mu$m.

(Comparative Example 1)

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades with the blade diameter of 5 cm) was prepared. Into this flask, 293.0 g of n-heptane was added as a hydrocarbon dispersion medium and 0.736 g of a maleic anhydride-modified ethylene/propylene copolymer (Mitsui Chemicals, Inc., High Wax 1105A) was added as a polymeric dispersant to obtain a mixture. The dispersant was dissolved by raising the temperature to 80°C while stirring the mixture, and then the mixture was cooled to 50°C.

Subsequently, 92.0 g of 80.5% by mass aqueous acrylic acid solution (acrylic acid: 1.03 mol) was added into a beaker having the internal volume of 300 mL as a water-soluble ethylenically unsaturated monomer. Subsequently, while cooling from the outside, 147.7 g of 20.9% by mass sodium hydroxide aqueous solution was added dropwise to perform 75 mol% of neutralization. Thereafter, 0.092 g of hydroxyethyl cellulose (Sumitomo Seika Chemicals Co., Ltd., HEC AW-15F) as a thickener, 0.0736 g (0.272 mmol) of potassium persulfate as a water-soluble radical polymerization initiator, and 0.010 g (0.057 mmol) of ethylene glycol diglycidyl ether as an internal crosslinking agent were added, and then dissolved therein to prepare a first stage aqueous solution.

Then, the above-mentioned first stage aqueous solution was added into the above-mentioned separable flask, and then stirred for 10 minutes. Thereafter, a surfactant solution obtained by heat-dissolving 0.736 g of sucrose stearic acid ester (surfactant, Mitsubishi-Chemical Foods Corporation, Ryoto Sugar Ester S-370) having HLB of 3 in 6.62 g of n-heptane was added into the separable flask. Then, the inside of the system was sufficiently replaced with nitrogen while stirring at the stirring speed of 550 rpm of the stirrer. Thereafter, the flask was immersed in a water bath at 70°C to raise the temperature, and polymerization was performed for 60 minutes to obtain a first stage polymerization slurry solution.

Subsequently, 128.8 g of 80.5% by mass aqueous acrylic acid solution (acrylic acid: 1.43 mol) was added into another beaker having the internal volume of 500 mL as a water-soluble ethylenically unsaturated monomer. Subsequently, while cooling from the outside, 159.0 g of 27% by mass sodium hydroxide aqueous solution was added dropwise to perform 75 mol% of neutralization. Thereafter, 0.090 g (0.334 mmol) of potassium persulfate as a water-soluble radical polymerization initiator, and 0.0116 g (0.067 mmol) of ethylene glycol diglycidyl ether as an internal crosslinking agent were added and then dissolved therein to prepare a second stage aqueous solution.

While stirring at the rotation speed of 1000 rpm of the stirrer, the inside of the above-mentioned separable flask was cooled to 26°C, and then the total amount of the above-mentioned second stage aqueous solution was added to the above-mentioned first stage polymerization slurry solution. Subsequently, after replacing the inside of the system with nitrogen for 30 minutes, the flask was immersed in a water bath at 70°C again to raise the temperature, and the polymerization reaction was performed for 60 minutes to obtain a second stage hydrogel-like polymer.

To the above-mentioned second stage hydrogel-like polymer, 0.265 g of 45% by mass diethylenetriamine pentasodium pentaacetate aqueous solution was added under stirring. Thereafter, the flask was immersed in an oil bath set at 125°C, and 256.1 g of water was extracted to the outside of the system while refluxing n-heptane by azeotropic distillation of n-heptane and water. Thereafter, 4.42 g of 2% by mass ethylene glycol diglycidyl ether aqueous solution (ethylene glycol diglycidyl ether: 0.507 mmol) was added into the flask as a surface crosslinking agent, and then the mixture was held at 83°C for 2 hours.

Thereafter, n-heptane and water were evaporated at 125°C by heating and dried to obtain a dried product. This dried product was passed through a sieve having the opening of 850 $\mu$m. Then, 0.2% by mass amorphous silica (Oriental Silicas Corporation, Tokusil NP-S) was mixed with the dried product based on the total mass of the dried product to obtain 230.8 g of water-absorbent resin particles.

Unless otherwise specified, the following various measurements and tests were carried out in an environment of the temperature of 25°C and the humidity of 60 $\pm$ 10%.

<Measurement of medium particle diameter and particle size distribution of water-absorbent resin particles>

The standard sieves were combined in the following order from the top: a sieve having the opening of 850 $\mu$m, a sieve having the opening of 500 $\mu$m, a sieve having the opening of 400 $\mu$m, a sieve having the opening of 300 $\mu$m, a sieve having the opening of 180 $\mu$m, and a tray.

50 g of the water-absorbent resin particles were put in the topmost sieve among the combined sieves, and classification was performed by shaking for 20 minutes using a Ro-tap sieve shaker (manufactured by Iida Seisakusho Co., Ltd., PAT. No. 531413). After classification, the mass of the water-absorbent resin particles remaining on each sieve was calculated as a mass percentage with respect to the total amount, and the particle size distribution was obtained. The relationship between the opening of the sieve and the integrated value of the mass percentage of the water-absorbent resin particles remaining on the sieve was plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass was obtained as the medium particle diameter [$\mu$m]. The results are shown in Table 1.

In addition, by using the above-mentioned sieve, the content of the particle diameter of 180 $\mu$m or less, the content of the particle diameter of more than 180 $\mu$m and 300 $\mu$m or less, the content of the particle diameter of more than 300 $\mu$m and 400 $\mu$m or less, the content of the particle diameter of more than 400 $\mu$m and 500 $\mu$m or less, and the content of the particle diameter of more than 500 $\mu$m and 850 $\mu$m or less, based on the total mass of the water-absorbent resin particles, were obtained. The results are shown in Table 1.

<Preparation of absorber>

Using an air flow type mixer (Padformer, manufactured by O-tec Co., Ltd.), 10 g of the water-absorbent resin particles and 10 g of pulverized pulp were uniformly mixed by air papermaking to prepare a sheet-shaped absorber having the size of 40 cm $\times$ 12 cm. Subsequently, in a state where the upper and lower sides of the absorber were sandwiched between two sheets of tissue paper having the same size as that of the absorber and having the basis weight of 16 g/m$^2$, the load of 196 kPa was applied to the entire body for 30 seconds and pressed to form an absorber.

<Falloff test>

(Absorber falloff test)

A test piece having the size of 14 cm × 12 cm was prepared by cutting off an end of the absorber symmetrically from the center and removing the tissue. After placing the test piece on a stack of a sieve having the inner diameter of 20 cm and the opening of 850 $\mu$m and a tray, vibration was applied to the test piece for 5 minutes using a Ro-tap type sieve shaker (PAT. No. 531413, manufactured by Iida Seisakusho Co., Ltd.). After carefully turning the test piece over, vibration was applied for 5 minutes in the same manner. A falloff product Z of the water-absorbent resin and the pulverized pulp that had fallen off from the test piece were collected in a tray. The contents of the water-absorbent resin and the pulverized pulp in the test piece after the test and the falloff product Z were calculated by the following content measurement method to obtain the content M11 of the water-absorbent resin and the content M12 of the pulverized pulp in the test piece after the test, and the content M21 of the water-absorbent resin and the content M22 of the pulverized pulp in the falloff product Z. Then, the falloff rate [mass%] of each component was calculated as follows. The results are shown in Table 1.

$$\text{Falloff rate of water-absorbent resin (SAP)} = M21/(M11 + M21) \times 100$$

$$\text{Falloff rate of pulp} = M22/(M12 + M22) \times 100$$

$$\text{Total falloff rate (Total)} = (M21 + M22)/(M11 + M12 + M21 + M22) \times 100$$

[Method for measuring the content of each component in the absorber]

{(1) Content proportion of water-absorbent resin}

A mass C [g] of the test piece or the falloff product Z, which was the object to be measured, was measured. Subsequently, the object to be measured was immersed in 8 L of ion-exchanged water and left for 30 minutes (after immersion, stirring was performed for 10 seconds to disperse the object to be measured). Subsequently, the object to be measured dispersed in ion-exchanged water was filtered through a standard sieve (inner diameter 20 cm, opening 75 $\mu$m, the same applies hereinafter) weighed in advance, and this standard sieve was filtrated by tilting for 30 minutes such that the inclination angle to the horizon was about 30 degrees. Subsequently, a mass D [g] of the object to be measured after water absorption was obtained by measuring the mass of the object to be measured together with the standard sieve.

Here, ion-exchanged water-absorbing ability A [g/g] of the water-absorbent resin and ion-exchanged water-absorbing ability B [g/g] of the pulp calculated by the methods shown in (2) and (3) below, and the content X [g] of the water-absorbent resin and the content Y [g] of the pulp satisfy the following formulae (I) and (II).

$$X + Y = C \qquad (\text{I})$$

$$AX + BY = D \qquad (\text{II})$$

By converting the formulae (I) and (II), the following formulae (III) and (IV) are obtained. Based on the formulae (III) and (IV), the content (X) of the water-absorbent resin and the content (Y) of the pulp in the object to be measured were calculated.

$$X = (D-BC)/(A-B) \quad (III)$$

$$Y = C-X \quad (IV)$$

{(2) Calculation of ion-exchanged water-absorbing ability of water-absorbent resin}

First, 0.5 g of the water-absorbent resin was put into 1000 mL of ion-exchanged water in a beaker to swell, and then left for 30 minutes. Subsequently, the water-absorbent resin after water absorption was transferred to a standard sieve weighed in advance, and then this standard sieve was filtrated by tilting for 30 minutes such that the inclination angle with respect to the horizon was about 30 degrees. Subsequently, a mass m1 [g] of the water-absorbent resin after swelling on the standard sieve was measured, and the ion-exchanged water-absorbing ability A [g/g] of the water-absorbent resin was obtained by the following formula (V).

$$A\,[g\,/\,g] = m1\,[g]/0.5\,[g] \quad (V)$$

{(3) Ion-exchanged water-absorbing ability of pulp}

First, 1.0 g of pulp was immersed in 1000 mL of ion-exchanged water in a beaker, and then left for 30 minutes. Subsequently, the pulp after water absorption was transferred to a standard sieve weighed in advance, and then this standard sieve was filtrated by tilting for 30 minutes such that the inclination angle with respect to the horizon was about 30 degrees. A mass m2 [g] of the pulp after water absorption on the standard sieve was measured and obtained as the ion-exchanged water-absorbing ability B [g/g] of the pulp.

(Falloff test of each particle diameter region)

By classifying the water-absorbent resin particles, particles having the particle diameter of 180 $\mu$m or less, particles having the particle diameter of more than 180 $\mu$m and 300 $\mu$m or less, particles having the particle diameter of more than 300 $\mu$m and 400 $\mu$m or less, particles having the particle diameter of more than 400 $\mu$m and 500 $\mu$m or less, and particles having the particle diameter of more than 500 $\mu$m and 850 $\mu$m or less were obtained each in an amount of 10 g or more. After preparing an absorber in the same manner as described above using 10 g of each particle classified in such a way and 10 g of pulverized pulp, a falloff rate of each particle diameter region was measured by the same method as in the above-mentioned falloff test for the entire absorber. The results are shown in Table 1.

<Calculation of yield>

A mass of the sheet-shaped absorber having the size of 40 cm × 12 cm prepared according to the <Preparation of absorber> was measured. Then, as the yield "mass%", a proportion of the mass of the absorber with respect to the charged amount (total amount of 20.0 g of 10.0 g of water-absorbent resin particles and 10.0 g of pulverized pulp) was calculated. The results are shown in Table 1.

[Table 1]

| | Medium particle diameter [μm] | Particle size distribution | | Falloff rate [% by mass] | | | Yield [% by mass] |
|---|---|---|---|---|---|---|---|
| | | | | SAP | Pulp | Total | |
| Example 1 | 370 | Total | - | 5.6 | 2.5 | 3.9 | 99.8 |
| | | 500 to 850 μm | 13% | 6.7 | 3.7 | 4.9 | |
| | | 400 to 500 μm | 24% | 5.4 | 2.2 | 3.6 | |
| | | 300 to 400 μm | 47% | 6.8 | 2.5 | 4.7 | |
| | | 180 to 300 μm | 11% | 5.7 | 1.9 | 3.7 | |
| | | 180 μm or less | 5% | 7.7 | 4.3 | 6.0 | |
| Example 2 | 400 | Total | - | 5.0 | 3.2 | 4.0 | 99.7 |
| | | 500 to 850 μm | 32% | 3.2 | 2.9 | 3.1 | |
| | | 400 to 500 μm | 18% | 4.2 | 2.3 | 3.3 | |
| | | 300 to 400 μm | 22% | 7.7 | 2.7 | 5.1 | |
| | | 180 to 300 μm | 21% | 7.7 | 3.6 | 5.5 | |
| | | 180 μm or less | 7% | 21.3 | 11.7 | 15.3 | |
| Example 3 | 424 | Total | - | 7.4 | 2.7 | 4.8 | 98.6 |
| | | 500 to 850 μm | 33% | 2.4 | 1.6 | 2.0 | |
| | | 400 to 500 μm | 23% | 3.7 | 1.9 | 2.7 | |
| | | 300 to 400 μm | 25% | 3.9 | 2.4 | 3.1 | |
| | | 180 to 300 μm | 13% | 5.4 | 0.9 | 2.6 | |
| | | 180 μm or less | 6% | 21.1 | 8.4 | 16.3 | |

(continued)

|  | Medium particle diameter [μm] | Particle size distribution | | Falloff rate [% by mass] | | | Yield [% by mass] |
|---|---|---|---|---|---|---|---|
|  |  |  |  | SAP | Pulp | Total |  |
| Comparative Example 1 | 353 | Total | - | 4.7 | 2.9 | 3.7 | 96.9 |
|  |  | 500 to 850 μm | 15% | 2.0 | 1.8 | 1.9 |  |
|  |  | 400 to 500 μm | 18% | 1.7 | 2.3 | 2.0 |  |
|  |  | 300 to 400 μm | 39% | 4.9 | 2.2 | 3.4 |  |
|  |  | 180 to 300 μm | 22% | 5.4 | 3.0 | 4.1 |  |
|  |  | 180 μm or less | 6% | 38.7 | 10.0 | 25.3 |  |

**Reference Signs List**

10: absorber, 10a: water-absorbent resin particles, 10b: fiber layer, 20a, 20b: core wrap, 30: liquid permeable sheet, 40: liquid impermeable sheet, 100: absorbent article.

**Claims**

1. An absorber comprising:

   water-absorbent resin particles; and
   a fibrous substance,
   wherein a medium particle diameter of the water-absorbent resin particles is 250 to 600 μm,
   the water-absorbent resin particles comprise small-diameter particles having a particle diameter of 180 μm or less, and
   a falloff rate of a mixture of the small-diameter particles and the fibrous substance when the mixture is subjected to shaking treatment for 10 minutes is 20% by mass or less.

2. The absorber according to claim 1,
   wherein a content of the small-diameter particles in the water-absorbent resin particles is 10% by mass or less.

3. The absorber according to claim 1 or 2,
   wherein the water-absorbent resin particles comprise a structural unit derived from at least one compound selected from the group consisting of (meth)acrylic acid and a salt thereof.

4. An absorbent article comprising:
   the absorber according to any one of claims 1 to 3.

5. The absorbent article according to claim 4, which is a diaper.

*Fig.1*

**INTERNATIONAL SEARCH REPORT**

| International application No. |
|---|
| PCT/JP2019/048794 |

A. CLASSIFICATION OF SUBJECT MATTER
C08F 20/06(2006.01)i; A61F 13/53(2006.01)i; B01J 20/26(2006.01)i; B01J 20/28(2006.01)i
FI: A61F13/53 300; B01J20/26 D; C08F20/06; B01J20/28 Z
According to International Patent Classification (IPC) or to both national classification and IPC

B. FIELDS SEARCHED

Minimum documentation searched (classification system followed by classification symbols)
C08F20/06; A61F13/53; B01J20/26; B01J20/28

Documentation searched other than minimum documentation to the extent that such documents are included in the fields searched
Published examined utility model applications of Japan        1922-1996
Published unexamined utility model applications of Japan      1971-2020
Registered utility model specifications of Japan              1996-2020
Published registered utility model applications of Japan      1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2018-94246 A (KAO CORP.) 21.06.2018 (2018-06-21) claims, paragraphs [0001]-[0072], fig. 1-2 | 1-5 |
| A | JP 2010-185029 A (SAN-DIA POLYMER LTD.) 26.08.2010 (2010-08-26) entire text, all drawings | 1-5 |

☐ Further documents are listed in the continuation of Box C.   ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>03 March 2020 (03.03.2020) | Date of mailing of the international search report<br>10 March 2020 (10.03.2020) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT
Information on patent family members

International application No.

PCT/JP2019/048794

| Patent Documents referred in the Report | Publication Date | Patent Family | Publication Date |
|---|---|---|---|
| JP 2018-94246 A | 21 Jun. 2018 | (Family: none) | |
| JP 2010-185029 A | 26 Aug. 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010185029 A
- WO 2018181565 A